# EUROPEAN PATENT APPLICATION

(11) **EP 4 675 279 A1**
(43) Date of publication of application: **07.01.2026**
(21) Application number: 25186909.5
(22) Date of filing: 02.07.2025
(51) Int. Cl.: G01N 33/86

(54) **PLATELET COUNT ASSAYS**

(30) Priority: 02.07.2024 US 202418761816
(71) Applicant: Instrumentation Laboratory Company, Bedford, MA 01730 (US)
(72) Inventor: BOTTENUS, Ralph, San Diego (US); NAIR, Prajeeda, San Diego (US); MOSKOWITZ, Keith, Westfield (US); MARTINEZ, Milena, Bedford (US)
(74) Representative: Müller-Boré & Partner Patentanwälte PartG mbB

(57) **Abstract**

Methods and materials that can be used to measure the concentration of platelets in whole blood samples (e.g., at the point of care) are provided herein.

## Description

### TECHNICAL FIELD

This document relates to methods and materials that can be used to quantify (e.g., measure the concentration of) platelets in whole blood samples. For example, this document relates to methods and materials that can be used to measure the concentration of platelets in whole blood samples at the point of care.

### BACKGROUND

Platelets, also referred to as thrombocytes, are small, colorless cell fragments in the blood that can form clots and stop or prevent bleeding. Platelet count tests can be performed to measure the number of platelets in the blood. A higher than normal platelet count is referred to as thrombocytosis, and can cause blood clots in the arms and legs that, if untreated, can lead to heart attack and/or stroke. A lower than normal platelet count is referred to as thrombocytopenia, and can cause excess bleeding after a cut or other injury that results in bleeding. In cases of excess bleeding, it can be desirable to determine whether the bleeding is the result of low platelet function activity or is due to a low number of platelets. Moreover, it may be desirable to rapidly determine platelet count/concentration at the point of care, in order to permit prompt determination of an appropriate response to excess bleeding, for example.

### SUMMARY

This document provides methods and materials that can be used to quantify (e.g., determine the concentration of) platelets in blood samples at the point of care. For example, this document provides solid substrate-antibody conjugates, as well as compositions containing the solid substrate-antibody conjugates. The solid substrate-antibody conjugates can be used in methods of determining platelet count/concentration at the point of care. This document also provides methods for making the solid substrate-antibody conjugates described herein, as well as methods for using the solid substrate-antibody conjugates. In some cases, the methods provided herein can include combining a blood sample (e.g., a whole blood sample) with a solid substrate-antibody conjugate, where the antibody has binding specificity for a platelet cell surface receptor (e.g., a glycoprotein receptor) such that platelets in the blood sample can bind to the solid substrate-antibody conjugates, and subjecting the blood sample/solid substrate-antibody combination to agglutination conditions. Agglutination of the conjugates can be measured as an indication of the number of platelets present in the blood sample.

In a first aspect, this document features a method for generating a composition containing a solid substrate-antibody conjugate. The method can include, or consist essentially of, (a) coupling an antibody to a solid substrate to generate a solid substrate-antibody conjugate; and (b) adding one or more agents to the solid substrate-antibody conjugate, wherein the one or more agents are selected from the group consisting of (i) protease inhibitors, (ii) inhibitors of platelet activation, (iii) stabilizing agents, and (iv) inhibitors of non-specific binding of the antibody. The method can include adding, to the solid substrate-antibody conjugate, one or more protease inhibitors. The method can include adding the one or more protease inhibitors to a concentration of about 0.1-1% (w/w). The method can include adding, to the solid substrate-antibody conjugate, one or more inhibitors of platelet activation. The one or more inhibitors of platelet activation can include one or more of tirofiban, cytochalasin D, and ethylenediaminetetraacetic acid (EDTA). The method can include adding tirofiban to a concentration of about 0.05 µg/mL to about 0.1 µg/mL. The method can include adding cytochalasin D to a concentration of about 10 to about 30 µM. The method can include adding EDTA to a concentration of about 1 mM to about 5mM. The method can include adding, to the solid substrate-antibody conjugate, one or more inhibitors of non-specific binding of the antibody. The one or more inhibitors of non-specific binding of the antibody can include an Fc block. The method can include adding the Fc block to a concentration of about 0.1% to about 1% (w/w). The solid substrate can be a bead. The bead can have a diameter of about 0.5 to about 10 µm, or can have a diameter of about 5.5 to about 6 µm. The bead can include latex. The bead can be a light absorbent bead. The light absorbent bead can be dyed with 5,5'-dichloro-11-diphenylamino-3,3'-diethyl-10,12-ethylenethiatricarbocyanine perchlorate (IR 140). The antibody can be an anti-platelet antibody. The antibody can be an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody. The coupling step can include (i) contacting a solid substrate having a polypeptide attached thereto with Traut's reagent to thiolate the polypeptide, thereby forming a solid substrate having a thiolated polypeptide attached thereto; (ii) contacting an antibody with a heterobifunctional crosslinker containing a maleimide reactive group, thereby forming a maleimide-activated antibody; and (iii) combining (1) the solid substrate having the thiolated polypeptide attached thereto and (2) the maleimide-activated antibody, wherein the maleimide-activated antibody becomes coupled to the solid substrate having the thiolated polypeptide attached thereto via a thiol-maleimide reaction, thereby forming the solid substrate-antibody conjugate. The polypeptide can be selected from the group consisting of bovine serum albumin (BSA) and fibrinogen. The heterobifunctional crosslinker can have a length of about 7 to 10 angstroms. The heterobifunctional crosslinker can be an amine-sulfhydryl crosslinker. The amine-sulfhydryl crosslinker can be sulfosuccinimidyl 4-(N-maleimidomethyl)cyclohexane-1-carboxylate (sulfo-SMCC) or m-maleimidobenzoyl-N-hydroxysuccinimide ester (MBS). The combining of (1) the solid substrate having the thiolated polypeptide attached thereto and (2) the maleimide-activated antibody can be at a molar ratio such that the thiolated polypeptide is present at about a 20- to 80-fold molar excess as compared to the maleimide-activated antibody. The method can further include lyophilizing the solid substrate-antibody conjugate.

In another aspect, this document features a composition that includes, consists essentially of, or consists of a solid substrate-antibody conjugate, wherein the solid substrate-antibody conjugate was generated using a method provided herein.

In another aspect, this document features a composition that includes, consists essentially of, or consists of (a) a solid substrate-antibody conjugate, in particular as described herein; and (b) one or more agents selected from the group consisting of (i) protease inhibitors, (ii) inhibitors of platelet activation, (iii) stabilizing agents, and (iv) inhibitors of non-specific binding of the antibody. The composition can contain one or more protease inhibitors. The composition can contain the one or more protease inhibitors at a concentration of about 0.1-1% (w/w). The composition can contain one or more inhibitors of platelet activation. The one or more inhibitors of platelet activation can include one or more of tirofiban, cytochalasin D, and ethylenediaminetetraacetic acid (EDTA). The composition can contain tirofiban at a concentration of about 0.05 µg/mL to about 0.1 µg/mL. The composition can contain cytochalasin D at a concentration of about 10 to about 30 µM. The composition can contain EDTA at a concentration of about 1 mM to about 5mM. The composition can contain one or more inhibitors of non-specific binding of the antibody. The one or more inhibitors of non-specific binding of the antibody can include an Fc block. The composition can contain the Fc block at a concentration of about 0.1% to about 1% (w/w). The solid substrate can be a bead. The bead can have a diameter of about 0.5 to about 10 µm, or can have a diameter of about 5.5 to about 6 µm. The bead can include latex. The bead can be a light absorbent bead. The light absorbent bead can be dyed with 5,5'-dichloro-11-diphenylamino-3,3'-diethyl-10, 12-ethylenethiatricarbocyanine perchlorate (IR 140). The antibody can be an anti-platelet antibody. The antibody can be an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody. The composition can be lyophilized.

In another aspect, this document features a method for quantifying platelets in a blood sample. The method can include, or consist essentially of (a) forming a mixture containing (i) a blood sample and (ii) a composition containing a solid substrate-antibody conjugate and one or more agents selected from the group consisting of protease inhibitors, inhibitors of platelet activation, stabilizing agents, and inhibitors of non-specific binding of the antibody, wherein the antibody has binding specificity for a platelet cell surface glycoprotein receptor, and incubating the mixture under conditions such that platelets in the blood sample bind to the antibody to generate solid substrate-antibody-platelet complexes; (b) subjecting the mixture to agglutination conditions, such that the solid substrate-antibody-platelet complexes agglutinate; (c) measuring agglutination of the solid substrate-antibody-platelet complexes; and (d) relating the measurement of step (c) to the number or concentration of platelets in the blood sample.

In still another aspect, this document features an article of manufacture designed for point of care use. The article of manufacture can include, consist essentially of, or consist of (a) a reagent for quantifying platelets in a blood sample, wherein the reagent includes a composition containing a solid substrate-antibody conjugate and one or more agents selected from the group consisting of protease inhibitors, inhibitors of platelet activation, stabilizing agents, and inhibitors of non-specific binding of the antibody, wherein the antibody has binding specificity for a platelet cell surface glycoprotein receptor; (b) a reagent for quantifying adenosine diphosphate (ADP) in the blood sample; (c) a reagent for quantifying thrombin receptor activating peptide (TRAP) in the blood sample; and (d) a reagent for quantifying arachidonic acid (AA) in the blood sample.

In another aspect, this document features an article of manufacture designed for point of care use, where the article of manufacture includes, consists essentially of, or consists of: (a) a reagent for quantifying platelets in a blood sample, wherein the reagent includes a composition containing a solid substrate-antibody conjugate and one or more agents selected from the group consisting of protease inhibitors, inhibitors of platelet activation, stabilizing agents, and inhibitors of non-specific binding of the antibody, wherein the antibody has binding specificity for a platelet cell surface glycoprotein receptor; (b) a reagent for quantifying ADP in the blood sample; (c) a reagent for quantifying TRAP in the blood sample; and (d) a reagent for quantifying collagen in the blood sample.

In yet another aspect, this document features a method for generating a solid substrate-antibody conjugate. The method can include, consist essentially of, or consist of (a) contacting a solid substrate having a polypeptide attached thereto with Traut's reagent to thiolate the polypeptide, thereby forming a solid substrate having a thiolated polypeptide attached thereto; (b) contacting an antibody with a heterobifunctional crosslinker containing a maleimide reactive group, thereby forming a maleimide-activated antibody; and (c) combining (i) the solid substrate having the thiolated polypeptide attached thereto and (ii) the maleimide-activated antibody, wherein the maleimide-activated antibody becomes coupled to the solid substrate having the thiolated polypeptide attached thereto via a thiol-maleimide reaction, thereby forming the solid substrate-antibody conjugate. The solid substrate can be a bead. The bead can have a diameter of about 0.5 to about 10 µm, or can have a diameter of about 5.5 to about 6 µm. The bead can include latex. The bead can be a light absorbent bead. The light absorbent bead can be dyed with 5,5'-dichloro-11-diphenylamino-3,3'-diethyl-10,12-ethylenethiatricarbocyanine perchlorate (IR 140). The antibody can be an anti-platelet antibody. The antibody can be an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody. The combining of (i) and (ii) can be at a molar ratio such that the thiolated polypeptide is present at about a 20- to 80-fold molar excess as compared to the maleimide-activated antibody. The method can further include lyophilizing the solid substrate-antibody conjugate.

In another aspect, this document features a solid substrate coupled to an antibody, wherein the solid substrate was coupled to the antibody using a method provided herein.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the description below. Other features, objects, and advantages of the invention will be apparent from the description and from the claims.

### DETAILED DESCRIPTION

This document provides methods and materials that can be used to determine the concentration and/or number of platelets in a blood sample (e.g., a whole blood sample) from a mammal (e.g., a human). For example, this document provides methods and materials that can be used to determine the concentration and/or number of platelets in a blood sample from a patient at the point of care. In some cases, the methods provided herein can be used in conjunction with an assay for platelet function. Having the ability to quantify platelets at the point of care can facilitate the expedient determination of whether a mammal's platelet concentration and/or count is low or high as compared to controls (e.g., platelet concentrations/counts in healthy mammals of the same species as the mammal being assessed).

In general, the methods and materials provided herein can be used to quantify platelets in a biological sample based on the interaction of receptors on the surface of platelets in a biological sample (e.g., a whole blood sample) with binding molecules (e.g., antibodies) attached to a solid support, and agglutination of the platelets that are associated with the solid support due to the interaction between the receptors and the antibodies.

In some cases, this document provides solid substrate-antibody conjugates in combination with other agents that can facilitate binding and agglutination. For example, this document provides compositions that contain (a) a solid substrate-antibody conjugate and (b) one or more protease inhibitors, one or more inhibitors of platelet activation, one or more stabilizing agents, and/or one or more inhibitors of non-specific binding of the antibody to molecules other than the platelet surface receptors. Without being bound by a particular mechanism of action, the one or more protease inhibitors, inhibitors of platelet activation, stabilizing agents, and/or inhibitors of non-specific antibody binding can reduce (e.g., prevent) platelet activation and non-specific clumping, and can promote sensitive measurement of platelet counts.

In some cases, a composition provided herein can contain (a) a solid substrate-antibody conjugate and (b) one or more protease inhibitors or one or more inhibitors of platelet activation or one or more stabilizing agents or one or more inhibitors of non-specific binding of the antibody. In some cases, a composition provided herein can contain (a) a solid substrate-antibody conjugate and (b) one or more protease inhibitors and one or more inhibitors of platelet activation. In some cases, a composition provided herein can contain (a) a solid substrate-antibody conjugate and (b) one or more protease inhibitors and one or more stabilizing agents. In some cases, a composition provided herein can contain (a) a solid substrate-antibody conjugate and (b) one or more protease inhibitors and one or more inhibitors of non-specific binding of the antibody. In some cases, a composition provided herein can contain (a) a solid substrate-antibody conjugate and (b) one or more inhibitors of platelet activation and one or more stabilizing agents. In some cases, a composition provided herein can contain (a) a solid substrate-antibody conjugate and (b) one or more inhibitors of platelet activation and one or more inhibitors of non-specific binding of the antibody. In some cases, a composition provided herein can contain (a) a solid substrate-antibody conjugate and (b) one or more protease inhibitors, one or more inhibitors of platelet activation, and one or more stabilizing agents. In some cases, a composition provided herein can contain (a) a solid substrate-antibody conjugate and (b) one or more protease inhibitors, one or more inhibitors of platelet activation, and one or more inhibitors of non-specific binding of the antibody. In some cases, a composition provided herein can contain (a) a solid substrate-antibody conjugate and (b) one or more protease inhibitors, one or more stabilizing agents, and one or more inhibitors of non-specific binding of the antibody. In some cases, a composition provided herein can contain (a) a solid substrate-antibody conjugate and (b) one or more protease inhibitors, one or more inhibitors of platelet activation, one or more stabilizing agents, and one or more inhibitors of non-specific binding of the antibody.

Any appropriate solid substrate can be used in the compositions provided herein. In some cases, the solid substrate can be in the form of a bead, film, membrane, tube, well, strip, or rod. For example, the solid substrate can be a bead. The solid substrate can contain any appropriate material. For example, the solid substrate can be made from a polymeric material such as latex, nitrocellulose, cellulose acetate, poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), polysaccharides such as dextrans and modified dextrans. In some cases, the solid substrate can be made from latex, which typically includes a water insoluble polymeric material. For example, latex can include a substituted polyethylene such as polystyrene-butadiene, polyacrylamide, polystyrene, polystyrene with carboxylic groups, polystyrene with amino groups, poly-acrylic acid, polymethacrylic acid, acrylonitrile-butadiene, styrene copolymers, polyvinyl acetate-acrylate, polyvinyl pyridine, and/or vinyl-chloride acrylate copolymers. For example, non-crosslinked polymers of styrene and carboxylated styrene or styrene functionalized with other active groups such as amino, hydroxyl, and/or halo groups can be used, or copolymers of substituted styrenes with dienes such as butadiene can be used.

In some cases, the solid substrate can include beads (e.g., latex beads). The beads can have any appropriate shape. For example, the beads can be generally spherical with uniform diameters. The beads can have any appropriate size. For example, the beads can have a maximum diameter of about 0.05 µm to about 100 µm (e.g., about 0.05 µm to about 0.1 µm, about 0.1 to about 0.5 µm, about 0.5 µm to about 1 µm, about 0.5 to about 5 µm, about 5 µm to about 10 µm, about 1 µm to about 2 µm, about 2 µm to about 3 µm, about 3 µm to about 4 µm, about 4 µm to about 5 µm, about 5 µm to about 6 µm, about 6 µm to about 10 µm, about 10 µm to about 20 µm, about 20 µm to about 30 µm, about 30 µm to about 50 µm, or about 50 µm to about 100 µm). In some cases, the beads can have a maximum diameter of about 5 µm to about 7 µm (e.g., about 5.5 µm to about 6 µm, or about 6 µm to about 6.5 µm). The beads can have any density. For example, the beads can have a density of about 0.7 g/mL to about 1.5 g/mL (e.g., about 0.7 g/mL to about 0.8 g/mL, about 0.8 g/mL to about 0.9 g/mL, about 0.9 g/mL to about 1.0 g/mL, about 1.0 g/mL to about 1.1 g/mL, about 1.1 g/mL to about 1.2 g/mL, about 1.2 g/mL to about 1.3 g/mL. about 1.3 g/mL to about 1.4 g/mL, or about 1.4 g/mL to about 1.5 g/mL).

In some cases, a solid substrate can include a light absorbent material. For example, a solid substrate can include beads (e.g., latex beads) that have been dyed with a light absorbent agent can be used. Any appropriate light absorbent agent can be used to dye a solid substrate. For example, latex beads can be dyed with 5,5'-dichloro-11-diphenylamino-3,3'-diethyl-10,12-ethylenethiatricarbocyanine perchlorate (IR 140). Any other appropriate dye can be used (e.g., BM-terphenyl, fluorescein, P-terphenyl, rhodamin dyes, sulforhodamin dyes, Exalite dyes, Oxazin dyes, Umbelliferon dyes, coumarin dyes, etc.). The light absorbent agent can provide a means by which to quantify platelets in a biological sample (e.g., a whole blood sample) after the biological sample is combined with a solid substrate-antibody conjugate provided herein.

In some cases, the surface of the solid substate can be hydrophilic or can be capable of being rendered hydrophilic. In some cases, the surface of the solid substrate can be polyfunctional or can be capable of being polyfunctionalized, and/or can be capable of binding to a platelet cell surface glycoprotein receptor-binding molecule (e.g., an antibody) through covalent or specific or non-specific non-covalent interactions. Such binding is indirect where non-covalent interactions are used and is direct where covalent interactions are employed. When a solid substate has functional groups on its surface through which a platelet-binding molecule can be attached, any appropriate functional group(s) can be used. Suitable functional groups include, without limitation, carboxylic acids, aldehydes, amino groups, cyano groups, ethylene groups, hydroxyl groups, and mercapto groups.

In some cases, a solid substrate can have binding molecules (e.g., polypeptides or other molecules) attached to its surface. The polypeptides or other molecules coated on the surface of the solid substrate can, for example, provide points of attachment for a platelet cell surface glycoprotein receptor-binding molecule (e.g., an antibody). Any suitable polypeptide or other molecule can be attached to the surface of a solid substrate. In some cases, bovine serum albumin (BSA) can be coated on the surface of a solid substrate described herein. Other examples of molecules that can be coated on the surface of a solid substrate provided herein include, without limitation, inert molecules such as fibrinogen and aminodextran, as well as collagen, Von Willebrand Factor, any potential ligand of a platelet receptor, Protein A, and protein G. A solid substate can have any appropriate amount of a binding molecule coupled to its surface. In general, the amount of the binding molecule employed is dependent on the size, affinity, and structural characteristics of the molecule. A binding molecule can be present in an amount of about 1 to about 100 (e.g., about 20 to about 100, about 30 to about 100, or about 90 to about 100) mole percent of the molecules present on the surface of the solid substrate.

The solid substrate-antibody conjugates used in the compositions and methods provided herein can include any appropriate antibody. An antibody is an immunoglobulin that specifically binds to and is thereby defined as complementary with a particular spatial and polar organization of another molecule, such as a platelet cell surface glycoprotein. The antibody can be monoclonal or polyclonal, and can include a complete immunoglobulin or fragment thereof. The immunoglobulin can be from any appropriate class and isotype, such as IgA, IgD, IgE, IgGl, IgG2a, IgG2b, IgG3, or IgM. Antibody fragments can be Fab, Fv and F(ab')2, or Fab' fragments. In addition, aggregates, polymers, and conjugates of immunoglobulins or their fragments can be used where appropriate, so long as binding affinity for the desired antigen is maintained.

The antibodies included in the substrate-antibody conjugates described herein can be commercially obtained or can be prepared by any appropriate technique. For example, antiserum containing polyclonal antibodies can be obtained using techniques that include immunization of an animal (e.g., a rabbit, guinea pig, or goat) with an appropriate immunogen and obtaining antisera from the blood of the immunized animal after an appropriate waiting period. Monoclonal antibodies can be generated by preparing continuous hybrid somatic cell lines and collecting the secreted protein. In general, samples of an immunogen preparation can be injected into an animal (e.g., a mouse) and, after a sufficient time, the animal can be sacrificed and spleen cells obtained. Alternatively, the spleen cells of a non-immunized animal can be sensitized to the immunogen *in vitro.* The spleen cell chromosomes encoding the base sequences for the desired immunoglobins can be compressed by fusing the spleen cells, generally in the presence of a non-ionic detergent (e.g., polyethylene glycol), with a myeloma cell line. The resulting cells, which include fused hybridomas, can be allowed to grow in a selective medium (e.g., HAT-medium), and the surviving immortalized cells can be grown in such medium using limiting dilution conditions. The cells can be grown in a suitable container (e.g., microtiter wells), and the cell supernatants can be screened for monoclonal antibodies having the desired specificity. In another approach for the preparation of antibodies, naturally occurring antibodies can be collected from cells by expressing, in the cells, nucleotide sequences encoding at the amino acid sequences required for specific binding of natural antibodies. In general, antibodies can be purified using techniques such as chromatography, including Protein A chromatography, Protein G chromatography, DEAE chromatography, or ABx chromatography.

The antibodies in the solid substrate-antibody conjugates can have specific binding affinity for a platelet cell surface glycoprotein receptor. That is, the antibodies can specifically recognize an epitope on a platelet cell surface glycoprotein receptor. Platelet cell surface glycoprotein receptors are carbohydrate-rich protein receptors on the surface of platelets. Platelet cell surface glycoprotein receptors include, for example, GPIbα and GPIIb/IIIa. These receptors can interact with binding molecules that cause aggregation of the receptors. The receptor binding molecules can be specific binding molecules or non-specific binding molecules. Examples of specific binding molecules include ligands for platelet cell surface glycoprotein receptors, such as von Willebrand factor, thrombin, and alboaggregins, as well as antibodies for platelet cell surface glycoprotein receptors (e.g., AN51, sz2, AK2, VMI6d, 6D1, HPL11, 4H12, TM-60, LJ1610, ES85, C34, API, MB45, which have specific binding affinity for the GPIbα receptor). Examples of non-specific binding molecules, which do not bind specifically to platelet cell surface glycoprotein receptors but can still cause aggregation of the receptors, include wheat germ agglutinin, concanavalin A, and poly-L-lysine.

In some cases, the antibody included in the conjugates described herein can be an anti-platelet glycoprotein Ib (GP1b, also referred to as GPIb or CD42b) antibody, an anti-platelet glycoprotein IIB/IIIa (GPIIB/IIIa) antibody, an anti-platelet glycoprotein Ib alpha chain (GPIbα) antibody, an anti-platelet glycoprotein IX (GPIX) antibody, or an anti-CD9 antibody. For example, the antibody included in the conjugates described herein can be an anti-GPIbα antibody. In some cases, the anti-GP1bα antibody can bind specifically to the N-terminal portion of the GPIbα receptor. Without being bound by a particular mechanism of action, such binding can be useful because the N-terminal portion of the GPIbα receptor is distanced away from the surface of the platelet cell surface (e.g., at least about 5 nm to about 30 nm from the cell surface). In some cases, however, the portion of the receptor recognized by the antibody may be less than about 5 nm from the platelet cell surface.

In some cases, the solid support-antibody conjugates described herein can include an antibody (e.g., an antibody directed against GPIbα) that inhibits von Willebrand factor binding to the receptor by at least about 50% (e.g., at least about 60%, at least about 70%, at least about 80%, or at least about 90%). In some cases, the antibody will not substantially interfere with functional aspects of the fibrinogen receptor, such that the antibody does not inhibit platelet agglutination induced by ADP or inhibited by IIb/IIIa antagonists. In some cases, the antibody can inhibit the binding of thrombin to GPIbα.

In some cases, an anti-GPIb antibody can be commercially obtained from, for example, Thermo Fisher Scientific (Waltham, MA) (e.g., catalog no. 14-0429-82, catalog no. PA5-109282, catalog no. MA5-51402, catalog no. MA5-44051, catalog no. MA5-43997, or catalog no. PA5-103118). In some cases, an anti-GPIb antibody can be obtained from AbCam (Cambridge, UK) (e.g., catalog no. ab210407, catalog no. ab134087, catalog no. ab240406, catalog no. ab252264, or catalog no. ab221213). In some cases, an anti-GPIb antibody can be obtained from BosterBio (Pleasanton, CA) (e.g., catalog no. M02073). In some cases, an anti-GPIb antibody can be obtained from Bioss Inc. (Woburn, MA) (e.g., catalog no. bsm-60017R).

In some cases, a composition provided herein can include one or more (e.g., two, three, four, five, or more than five) protease inhibitors. For example, a composition provided herein can contain a cocktail of two or more protease inhibitors. The one or more protease inhibitors can, for example, reduce or prevent protein or enzyme degradation by proteolytic agents. Any appropriate protease inhibitor can be included in a composition provided herein. Examples of suitable protease inhibitors include, without limitation, aprotinin, bestatin, calpain inhibitor I and II, chymostatin, E-64, leupeptin alpha-2-macroglobulin, pepstatin, phenylmethanesulfonyl fluoride (PMSF), tosyllysine chloromethyl ketone hydrochloride (TLCK), ethylenediaminetetraacetic acid (EDTA), and trypsin inhibitors, and any combination thereof. In some cases, a cocktail of protease inhibitors can be used. Such cocktails can, in some cases, be commercially obtained (e.g., from Thermo Fisher Scientific, Sigma Aldrich, In some cases, each protease inhibitor that is included in a composition provided herein can be present in the composition in an amount of about 0.01 wt% to about 5 wt% (e.g., about 0.01 wt% to about 0.05 wt%, about 0.05 wt% to about 0.1 wt%, about 0.1 wt% to about 0.5 wt%, about 0.1 wt% to about 1 wt%, about 0.5 wt% to about 1 wt%, about 1 wt% to about 2 wt%, about 2 wt% to about 3 wt%, or about 3 wt% to about 5 wt%). In some cases, a composition provided herein can include two or more protease inhibitors, where the total amount of the two or more protease inhibitors in the composition is about 0.01 wt% to about 5 wt% (e.g., about 0.01 wt% to about 0.05 wt%, about 0.05 wt% to about 0.1 wt%, about 0.1 wt% to about 0.5 wt%, about 0.1 wt% to about 1 wt%, about 0.5 wt% to about 1 wt%, about 1 wt% to about 2 wt%, about 2 wt% to about 3 wt%, or about 3 wt% to about 5 wt%).

In some cases, a composition provided herein can include one or more inhibitors of platelet activation. The one or more inhibitors of platelet activation can reduce or prevent platelet activation (e.g., by a detecting antibody used in the assays described herein), which could lead to erroneous measurements. Any appropriate inhibitor of platelet activation can be included. Examples of suitable inhibitors of platelet activation include, without limitation, tirofiban, cytochalasin D, and EDTA. In some cases, each inhibitor of platelet activation that is included in a composition provided herein can be present in the composition at a concentration of about 0.005 µg/mL to about 1 µg/mL (e.g., about 0.005 µg/mL to about 0.01 µg/mL, about 0.01 µg/mL to about 0.05 µg/mL, about 0.5 µg/mL to about 0.1 µg/mL, about 0.1 µg/mL to about 0.5 µg/mL, or about 0.5 µg/mL to about 1 µg/mL). In some cases, the one or more inhibitors of platelet activation that are included in a composition provided herein can be present in the composition at a total concentration of about 0.005 µg/mL to about 1 µg/mL (e.g., about 0.005 µg/mL to about 0.01 µg/mL, about 0.01 µg/mL to about 0.05 µg/mL, about 0.5 µg/mL to about 0.1 µg/mL, about 0.1 µg/mL to about 0.5 µg/mL, or about 0.5 µg/mL to about 1 µg/mL). In some cases, a composition provided herein can contain cytochalasin D, where the cycochalasin D is present in the composition at a concentration of about 1 µM to about 100 µM (e.g., about 1 µM to about 5 µM, about 5 µM to about 10 µM, about 10 µM to about 20 µM, about 10 µM to about 30 µM, about 20 µM to about 30 µM, about 30 µM to about 50 µM, or about 50 µM to about 100 µM). In some cases, a composition provided herein can contain EDTA at a concentration of about 0.1 mM to about 50 mM (e.g., about 0.1 mM to about 0.5 mM, about 0.5 mM to about 1 mM, about 1 mM to about 5 mM, about 1 mM to about 10 mM, about 5 mM to about 10 mM, about 10 mM to about 25 mM, or about 25 mM to about 50 mM).

In some cases, a composition provided herein can include one or more (e.g., two, three, four, five, or more than five) stabilizers. Any appropriate stabilizer can be included. In some cases, a stabilizer also can be an inhibitor of platelet activation. An example of a suitable stabilizer is EDTA (which also can inhibit platelet activation). Each stabilizer that is included in a composition provided herein can be present in the composition in any appropriate amount. In some cases, the one or more protease inhibitors that are included in a composition provided herein can be present in the composition at a total concentration of about 0.01 wt% to about 5 wt%. In some cases, a composition provided herein can contain EDTA at a concentration of about 0.1 mM to about 50 mM (e.g., about 0.1 mM to about 0.5 mM, about 0.5 mM to about 1 mM, about 1 mM to about 5 mM, about 1 mM to about 10 mM, about 5 mM to about 10 mM, about 10 mM to about 25 mM, or about 25 mM to about 50 mM).

In some cases, a composition provided herein can include one or more inhibitors of non-specific binding of the antibody. The one or more inhibitors of non-specific binding of the antibody can prevent non-specific antibody binding to platelets or to lymphocyte Fc receptors. Any appropriate inhibitor of non-specific binding of the antibody can be included. Examples of suitable inhibitors of non-specific antibody binding include, without limitation, Fc blockers (e.g., Fc Receptor Binding Inhibitor polyclonal antibodies such as those available from Thermo Fisher Scientific/Invitrogen, BD Biosciences, and Bio-Rad Laboratories, Inc.). Each of the one or more inhibitors of non-specific antibody binding that are included in a composition provided herein can be present in the composition at a concentration of about 0.01 wt% to about 10 wt% (e.g., about 0.01 wt% to about 0.05 wt%, about 0.05 wt% to about 0.1 wt%, about 0.1 wt% to about 0.5 wt%, about 0.1 wt% to about 1 wt%, about 0.5 wt% to about 1 wt%, about 1 wt% to about 2 wt%, about 2 wt% to about 3 wt%, about 3 wt% to about 5 wt%, or about 5 wt% to about 10 wt%). In some cases, the one or more inhibitors of non-specific antibody binding that are included in a composition provided herein can be present in the composition at a total concentration of about 0.01 wt% to about 10 wt% (e.g., about 0.01 wt% to about 0.05 wt%, about 0.05 wt% to about 0.1 wt%, about 0.1 wt% to about 0.5 wt%, about 0.1 wt% to about 1 wt%, about 0.5 wt% to about 1 wt%, about 1 wt% to about 2 wt%, about 2 wt% to about 3 wt%, about 3 wt% to about 5 wt%, or about 5 wt% to about 10 wt%).

In some cases, the compositions provided herein can contain a solid substrate-antibody conjugate and two or more protease inhibitors, tirofiban, cytochalasin, EDTA, and Fc block. For example, a composition provided herein can contain a about 0.1 wt% to about 1 wt% of a protease inhibitor cocktail, about 0.05 µg/mL to about 0.1 µg/mL tirofiban, about 10 µM to about 30 µM cytochalasin D, about 1 mM to about 5 mM EDTA, and about 0.1 wt% to about 1 wt% Fc block.

The compositions provided herein can have any appropriate form. For example, the compositions provided herein can be lyophilized. In some cases, the compositions provided herein can be suspensions or aqueous solutions.

This document also provides methods for making the compositions provided herein. In general, the methods can include coupling an antibody to a solid substrate to generate a solid substrate-antibody conjugate, and adding one or more agents to the solid substrate-antibody conjugate, where the one or more agents can include one or more protease inhibitors, one or more inhibitors of platelet activation, one or more stabilizing agents, and/or one or more inhibitors of non-specific binding of the antibody.

Any appropriate method can be used to couple an antibody to a solid substrate (e.g., a latex bead coated with BSA). In some cases, a solid substrate (e.g., a latex bead) coated with a polypeptide (e.g., BSA) can be contacted with Traut's reagent in order to thiolate the polypeptide. The method also can include contacting an antibody (e.g., an anti-GPIbα antibody) with a heterobifunctional crosslinker having a maleimide reactive group and a second reactive group that can be coupled to the antibody, to generate a maleimide-activated antibody. Any appropriate heterobifunctional crosslinker can be used. Examples of heterobifunctional crosslinkers containing a maleimide reactive group and a second reactive group that can be coupled to an antibody include, without limitation, amine-sulfhydryl crosslinkers such as sulfosuccinimidyl 4-(N-maleimidomethyl)cyclohexane-1-carboxylate (sulfo-SMCC), m-maleimidobenzoyl-N-hydroxysuccinimide ester (MBS), 6-maleimidocaproic acid sulfo-N-succinimidyl ester, and maleimidoacetic acid N-hydroxysuccinimide ester. The heterobifunctional crosslinker can have any appropriate length. For example, the heterobifunctional crosslinker can have a length of about 5 to about 15 angstroms (e.g., about 5 to about 7 angstroms, about 7 to 10 angstroms, about 10 to about 12 angstroms, or about 12 to about 15 angstroms).

The solid substrate coated with the thiolated polypeptide then can be combined with the maleimide-activated antibody under conditions such that the maleimide-activated antibody becomes coupled to the thiolated polypeptide attached to the solid substrate, via a thiol-maleimide reaction. Any appropriate amounts of the solid substrate coated with the thiolated polypeptide and the maleimide-activated antibody can be used. For example, the solid substrate coated with the thiolated polypeptide and the maleimide-activated antibody can be combined at a molar ratio such that the thiolated polypeptide is present at about a 10- to 150-fold (e.g., 10- to 20-fold, 20- to 30-fold, 20- to 50-fold, 20-to 80-fold, 30- to 50-fold, 50 to 70-fold, or 70- to 100-fold) molar excess as compared to the maleimide-activated antibody.

It is to be noted that other methods can be used to couple an antibody to a solid substrate (e.g., a solid substrate coated with an antibody-binding molecule as described herein). For example, polymerized acrylonitrile beads having N-hydroxysuccinimide ester groups on their surface can be used. The N-hydroxysuccinimide ester groups can allow for coupling of the N-terminus of an antibody to the surface of the bead. **In** some cases, carboxylated polystyrene beads with surface carboxyl groups can be coupled to the N-terminus of an antibody by means of, for example, carbodiimide coupling or N-hydroxysuccinimide coupling. The beads can be colored or dyed to make the results of the agglutination reaction easier to interpret.

A method for making a composition provided herein can further include adding, to a solid substrate-antibody conjugate, one or more additional agents. For example, a method can include adding, to a solid substrate-antibody conjugate, one or more protease inhibitors, one or more inhibitors of platelet activation, one or more stabilizing agents, and/or one or more inhibitors of non-specific binding of the antibody. Examples of such agents are described above. Further, these agents can be added in any appropriate combination, and at any appropriate amount, as described above.

In some cases, a method for making a composition containing a solid substrate-antibody conjugate can further include lyophilizing the solid substrate-antibody conjugate composition (e.g., after combining the conjugate with one or more protease inhibitors, one or more inhibitors of platelet activation, one or more stabilizing agents, and/or one or more inhibitors of non-specific binding of the antibody).

This document also provides methods for using the compositions provided herein to quantify platelets in biological samples (e.g., a blood samples). In general, the methods can include:
(a) combining a platelet-containing biological sample (e.g., a blood sample) with a composition provided herein that contains a solid substrate-antibody conjugate and one or more protease inhibitors, inhibitors of platelet activation, stabilizing agents, and/or inhibitors of non-specific binding of the antibody, where the antibody has binding specificity for a platelet cell surface glycoprotein receptor (e.g., an anti-GPIbα antibody), such that platelets in the biological sample bind to the antibody to yield solid substrate-antibody-platelet complexes;
(b) subjecting the solid substrate-antibody-platelet complexes to agglutination conditions such that the solid substrate-antibody-platelet complexes agglutinate;
(c) measuring agglutination of the solid substrate-antibody complex; and
(d) relating the measurement of (c) to the number or concentration of platelets in the biological sample.

Any appropriate platelet-containing sample can be assessed using the methods and materials provided herein. For example, a bodily fluid such as whole blood can be assessed using the methods and materials provided herein. In some cases, a platelet-containing blood fraction (e.g., platelet-rich plasma) can be assessed using the methods and materials provided herein. Any other appropriate platelet-containing sample can be used. In some cases, a sample can be an unprocessed sample obtained directly from a mammal (e.g., a human). In some cases, a sample can be preprocessed or prepared in any appropriate liquid medium (e.g., an aqueous medium). Preprocessing can include, for example, treatment with an anticoagulant. In some cases, an untreated whole blood sample can be used, or a whole blood sample treated with an anticoagulant can be used.

A platelet-containing sample that is assessed using the methods and materials provided herein can have any appropriate volume. Typically, the amount of the sample can depend on the nature of the sample. For example, for whole blood, a sample size of about 30 µL to about 300 µL (e.g., about 50 µL to about 250 µL, about 100 µL to about 200 µL, or about 150 µL) can be used.

Before being combined with a solid substrate-antibody conjugate in the methods provided herein, blood can be drawn from a mammal (e.g., a human patient) by any appropriate technique for quantification of platelets. For example, blood can be drawn into a closed vessel such as an evacuated tube, in particular a vacuum-based tube, for example a VACUTAINER^{®} tube, to protect the individual who is drawing the blood from blood-borne infectious agents (e.g., hepatitis B virus or HIV virus) that may be present in the blood. If the blood is to be mixed with an anticoagulant, the blood may be drawn into a tube containing anticoagulant or by adding anticoagulant to drawn blood. Suitable anticoagulants include, without limitation, EDTA, citrate, oxalate, heparin, hirudin, and other antithrombin agents.

The methods provided herein can include combining a platelet-containing biological sample (e.g., a blood sample) with a composition provided herein (a composition that contains a solid substrate-antibody conjugate and one or more protease inhibitors, inhibitors of platelet activation, stabilizing agents, and/or inhibitors of non-specific binding of the antibody, where the antibody has binding specificity for a platelet cell surface glycoprotein receptor (GPIbα) in an aqueous medium. In some cases, the medium can contain one or more polar cosolvents. For example, the medium can contain one or more oxygenated organic solvents of from 1-6 carbon atoms (e.g., from 1-4 carbon atoms), such as alcohols and/or ethers. When included in the medium, such cosolvents can be are present in an amount less than about 70 wt% (e.g., less than about 50 wt%, or less than about 30 wt%). In some cases, the assay medium can contain one or more buffers, stabilizers for the assay medium and the assay components, surfactants (e.g., non-ionic surfactants), and/or binding enhancers (e.g., polyalkylene glycols).

The assay medium can have any appropriate pH. In some cases, the medium can have a pH in the range of about 2 to about 11 (e.g., about 4 to about 9). The pH can be selected to maximize the extent of binding between the platelet cell surface glycoprotein receptor and the solid substrate-antibody conjugate employed in the method, thereby maximizing the extent of agglutination achieved. Any appropriate buffer(s) can be used to achieve the desired pH and to maintain the pH during the assay procedure. Examples of appropriate buffers include, without limitation, 2-[4-(2-hydroxyethyl)piperazin-1-yl] ethanesulfonic acid (HEPES), borate, phosphate, carbonate, Tris, and barbital. In some cases, HEPES can be included in the assay medium at a concentration of about 0.05 M to about 0.001 M (e.g., about 0.01 M). The buffer(s) also can maintain the salt concentration of the liquid within a range suitable for agglutination. Thus, the buffer can contain a concentration of one or more salts (e.g., sodium chloride) that maintain the electrolytic balance of the blood sample within a range suitable for agglutination. Any appropriate concentration of sodium chloride can be included in the buffer (e.g., about 0.1 M and about 0.2 M (e.g., about 0.15 M). Other salts that may be present include, without limitation, calcium chloride and magnesium chloride.

The order of addition of the various reagents may be varied. In some cases, the biological sample can be combined with the liquid medium before addition of the solid substate-antibody conjugate. In other cases, however, the biological sample and the solid substrate-antibody conjugate can be combined with the liquid medium substantially simultaneously.

After the biological sample and the solid substrate-antibody conjugates are combined in an appropriate medium, the medium can be subjected to agglutination conditions. For example, moderate temperatures can be employed for carrying out the method. The temperature can be held constant or can vary during the course of the agglutination period. The temperature employed can be at least about 25°C, but can range from about 10°C to about 80°C (e.g., from about 10°C to about 20°C, from about 15°C to about 45°C, from about 20°C to about 40°C, from about 30°C to about 40°C, or about 37°C). The number of platelets to be determined can vary, but typically is between about 10,000 platelets/µL and about 450,000 platelets/µL (e.g., about 25,000 platelets/µL to about 200,000 platelets/µL). It is to be noted that a count of less than about 10,000 platelets/µL in a sample from a human can indicate the need for a blood transfusion.

The agglutination can be carried out for any appropriate time period. For example, agglutination can be carried for about 30 seconds to about 1 hour (e.g., from about 10 seconds to about 2 minutes, from about 20 seconds to about 1 minute, from about 2 minutes to about 5 minutes, from about 5 minutes to about 10 minutes, from about 10 minutes to about 30 minutes, or from about 30 minutes to about 1 hour). In general, the time period for the reaction can be sufficient to permit substantial agglutination of the solid substrate-antibody-platelet complexes. In some cases, the medium can be agitated during the reaction period to facilitate binding of the antibody to the platelet cell surface glycoprotein receptor. Agitation can be achieved mechanically, such as by placing the reaction vessel on a rocker. The vessel may be rocked for the entire reaction period or for a shorter period as long as the basic objective of agglutination is achieved. In some cases, the reaction vessel can be rocked at about 6 cpm to about 900 cpm (e.g., about 300 cpm), controlled to about 0.1% of the preferable cpm. In some cases, the reaction medium can be agitated manually, such as by mixing with a mixing rod or by rotating on a glass slide. The time period for manual agitation can be substantially the same as that for mechanical agitation.

After incubation for an appropriate length of time, agglutination of the solid substrate-antibody-platelet complexes can be measured as an indication of the number of platelet cell surface glycoprotein receptors present in the medium. In some cases, the presence of agglutination can be determined visually by observing clumping of the complexes, which indicates agglutination. Alternatively, as described above, the solid substrate can be dyed with a light absorbent agent, which can aid in visualizing agglutination or clumping of the complexes. In some cases, dyes that absorb in the infrared range can be used. In some cases, the extent of agglutination can be measured spectrophotometrically, turbidimetrically, or nephelometrically.

The level of agglutination is an indication of the number of receptors present, which can be directly related to the number of platelets in the sample. In some cases, the level of agglutination can be compared against a control or a standard of known platelet number to determine the number of platelets in the sample. For example, the result can be compared to a calibrator, which can be performed concomitantly, performed previously, or provided as a standard curve.

In some cases, the methods provided herein can be used for rapid monitoring of an individual (e.g., a human patient) during a medical procedure, or to determine whether an individual is ready to undergo a medical procedure. This type of monitoring is referred to as "bedside monitoring" or "point of care monitoring." As described herein, the methods and materials provided herein offer advantages over platelet enumeration methods that require sophisticated instrumentation. The presently described methods can be carried out without the need for such instrumentation. Thus, the determination of platelet count can be conducted at the bedside. If the determination indicates a low platelet count, the medical procedure may be postponed until the situation has been corrected. For example, platelet transfusions may be administered immediately so that the medical procedure (e.g., an invasive surgery) may be carried out as soon as possible and excessive bleeding may be avoided. In some cases, the platelet count can be monitored during the platelet transfusions until a desirable level is achieved.

This document also provides articles of manufacture containing materials described herein. For example, this document provides kits for determining the number of platelets in a sample. The kits can include, in packaged combination, (a) a solid substrate to which is bound an antibody to a platelet cell surface glycoprotein receptor (e.g., the GPIbα subunit of the GPIb/lx-v receptor), and (b) a buffered medium. The kit also can include additional reagents for carrying out the methods described herein, and/or additional reagents for carrying out an assay for platelet function. The kits also can include a sample collection container and/or a device for carrying out the methods described herein and/or a platelet function assay. The relative amounts of reagents can vary to provide for concentrations in solution of the reagents that substantially optimize the sensitivity of a determination. Where appropriate, the reagents can be placed in an airtight package in order to maintain the activity of any reagents. The package can be, for example, a bag or pouch fabricated from a material that is substantially non-permeable to moisture. Such materials include, without limitation, plastic, aluminum foil, and the like. For blood samples, the kit also can include an article for piercing the skin, disinfectant, and/or sterilizing pads. It will be appreciated that additional articles can be included in the kits depending on type of sample being collected. In some cases, a kit also can include one or more calibrators and/or standards.

In some cases, this document provides an article of manufacture designed for point of care use in quantifying platelets in a biological sample, as well as for quantifying other components (e.g., polypeptides) in the sample.

For example, an article of manufacture designed for point of care use can include:
(a) a reagent for quantifying platelets in a blood sample, where the reagent includes a composition containing a solid substrate-antibody conjugate and one or more protease inhibitors, inhibitors of platelet activation, stabilizing agents, and/or inhibitors of non-specific binding of the antibody, where the antibody has binding specificity for a platelet cell surface glycoprotein receptor;
(b) a reagent for quantifying adenosine diphosphate (ADP) in the blood sample;
(c) a reagent for quantifying thrombin receptor activating peptide (TRAP) in the blood sample; and
(d) a reagent for quantifying arachidonic acid (AA) in the blood sample.

In another example, an article of manufacture designed for point of care use can include:
(a) a reagent for quantifying platelets in a blood sample, where the reagent includes a composition containing a solid substrate-antibody conjugate and one or more protease inhibitors, inhibitors of platelet activation, stabilizing agents, and/or inhibitors of non-specific binding of the antibody, where the antibody has binding specificity for a platelet cell surface glycoprotein receptor;
(b) a reagent for quantifying ADP in the blood sample;
(c) a reagent for quantifying TRAP in the blood sample; and
(d) a reagent for quantifying collagen in the blood sample.

### Exemplary Embodiments

Embodiment 1 is a method for generating a composition comprising a solid substrate-antibody conjugate, wherein the method comprises (a) coupling an antibody to a solid substrate to generate a solid substrate-antibody conjugate; and (b) adding one or more agents to the solid substrate-antibody conjugate, wherein the one or more agents are selected from the group consisting of (i) protease inhibitors, (ii) inhibitors of platelet activation, (iii) stabilizing agents, and (iv) inhibitors of non-specific binding of the antibody.

Embodiment 2 is the method of embodiment 1, wherein the method comprises adding, to the solid substrate-antibody conjugate, one or more protease inhibitors.

Embodiment 3 is the method of embodiment 2, wherein the method comprises adding the one or more protease inhibitors to a concentration of about 0.1-1% (w/w).

Embodiment 4 is the method of any one of embodiments 1 to 3, wherein the method comprises adding, to the solid substrate-antibody conjugate, one or more inhibitors of platelet activation.

Embodiment 5 is the method of embodiment 4, wherein the one or more inhibitors of platelet activation comprise one or more of tirofiban, cytochalasin D, and ethylenediaminetetraacetic acid (EDTA).

Embodiment 6 is the method of embodiment 5, wherein the method comprises adding the tirofiban to a concentration of about 0.05 µg/mL to about 0.1 µg/mL.

Embodiment 7 is the method of embodiment 5 or embodiment 6, wherein the method comprises adding the cytochalasin D to a concentration of about 10 to about 30 µM.

Embodiment 8 is the method of any one of embodiments 5 to 7, wherein the method comprises adding the EDTA to a concentration of about 1 mM to about 5mM.

Embodiment 9 is the method of any one of embodiments 1 to 8, wherein the method comprises adding, to the solid substrate-antibody conjugate, one or more inhibitors of non-specific binding of the antibody.

Embodiment 10 is the method of embodiment 9, wherein the one or more inhibitors of non-specific binding of the antibody comprise an Fc block.

Embodiment 11 is the method of embodiment 10, wherein the method comprises adding the Fc block to a concentration of about 0.1% to about 1% (w/w).

Embodiment 12 is the method of any one of embodiments 1 to 11, wherein the solid substrate is a bead.

Embodiment 13 is the method of embodiment 12, wherein the bead has a diameter of about 0.5 to about 10 µm.

Embodiment 14 is the method of embodiment 12, wherein the bead has a diameter of about 5.5 to about 6 µm.

Embodiment 15 is the method of any one of embodiments 12 to 14, wherein the bead comprises latex.

Embodiment 16 is the method of any one of embodiments 12 to 15, wherein the bead is a light absorbent bead.

Embodiment 17 is the method of embodiment 16, wherein the light absorbent bead is dyed with 5,5'-dichloro-11-diphenylamino-3,3'-diethyl-10,12-ethylenethiatricarbocyanine perchlorate (IR 140).

Embodiment 18 is the method of any one of embodiments 1 to 17, wherein the antibody is an anti-platelet antibody.

Embodiment 19 is the method of embodiment 18, wherein the antibody is an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody.

Embodiment 20 is the method of any one of embodiments 1 to 19, wherein the coupling step comprises (i) contacting a solid substrate comprising a polypeptide attached thereto with Traut's reagent to thiolate the polypeptide, thereby forming a solid substrate comprising a thiolated polypeptide attached thereto; (ii) contacting an antibody with a heterobifunctional crosslinker comprising a maleimide reactive group, thereby forming a maleimide-activated antibody; and (iii) combining (1) the solid substrate comprising the thiolated polypeptide attached thereto and (2) the maleimide-activated antibody, wherein the maleimide-activated antibody becomes coupled to the solid substrate comprising the thiolated polypeptide attached thereto via a thiol-maleimide reaction, thereby forming the solid substrate-antibody conjugate.

Embodiment 21 is the method of embodiment 20, wherein the polypeptide is selected from the group consisting of bovine serum albumin (BSA) and fibrinogen.

Embodiment 22 is the method of embodiment 20 or embodiment 21, wherein the heterobifunctional crosslinker has a length of about 7 to 10 angstroms.

Embodiment 23 is the method of any one of embodiments 20 to 22, wherein the heterobifunctional crosslinker is an amine-sulfhydryl crosslinker.

Embodiment 24 is the method of embodiment 23, wherein the amine-sulfhydryl crosslinker is sulfosuccinimidyl 4-(N-maleimidomethyl)cyclohexane-1-carboxylate (sulfo-SMCC) or m-maleimidobenzoyl-N-hydroxysuccinimide ester (MBS).

Embodiment 25 is the method of any one of embodiments 19 to 24, wherein the combining of (1) the solid substrate comprising the thiolated polypeptide attached thereto and (2) the maleimide-activated antibody is at a molar ratio such that the thiolated polypeptide is present at about a 20- to 80-fold molar excess as compared to the maleimide-activated antibody.

Embodiment 26 is the method of any one of embodiments 1 to 25, wherein the method further comprises lyophilizing the solid substrate-antibody conjugate.

Embodiment 27 is a composition comprising a solid substrate-antibody conjugate, wherein the solid substrate-antibody conjugate was generated using the method of any one of embodiments 1 to 26.

Embodiment 28 is a composition comprising: a solid substrate-antibody conjugate; and one or more agents selected from the group consisting of (i) protease inhibitors, (ii) inhibitors of platelet activation, (iii) stabilizing agents, and (iv) inhibitors of non-specific binding of the antibody.

Embodiment 29 is the composition of embodiment 28, wherein the composition comprises one or more protease inhibitors.

Embodiment 30 is the composition of embodiment 29, wherein the composition comprises the one or more protease inhibitors at a concentration of about 0.1-1% (w/w).

Embodiment 31 is the composition of any one of embodiments 28 to 30, wherein the composition comprises one or more inhibitors of platelet activation.

Embodiment 32 is the composition of embodiment 31, wherein the one or more inhibitors of platelet activation comprise one or more of tirofiban, cytochalasin D, and ethylenediaminetetraacetic acid (EDTA).

Embodiment 33 is the composition of embodiment 32, wherein the composition comprises the tirofiban at a concentration of about 0.05 µg/mL to about 0.1 µg/mL.

Embodiment 34 is the composition of embodiment 32 or embodiment 33, wherein the composition comprises the cytochalasin D at a concentration of about 10 to about 30 µM.

Embodiment 35 is the composition of any one of embodiments 32 to 34, wherein the composition comprises the EDTA at a concentration of about 1 mM to about 5mM.

Embodiment 36 is the composition of any one of embodiments 28 to 35, wherein the composition comprises one or more inhibitors of non-specific binding of the antibody.

Embodiment 37 is the composition of embodiment 36, wherein the one or more inhibitors of non-specific binding of the antibody comprise an Fc block.

Embodiment 38 is the composition of embodiment 37, wherein the composition comprises the Fc block at a concentration of about 0.1% to about 1% (w/w).

Embodiment 39 is the composition of any one of embodiments 28 to 38, wherein the solid substrate is a bead.

Embodiment 40 is the composition of embodiment 39, wherein the bead has a diameter of about 0.5 to about 10 µm.

Embodiment 41 is the composition of embodiment 39, wherein the bead has a diameter of about 5.5 to about 6 µm.

Embodiment 42 is the composition of any one of embodiments 39 to 41, wherein the bead comprises latex.

Embodiment 43 is the composition of any one of embodiments 39 to 42, wherein the bead is a light absorbent bead.

Embodiment 44 is the composition of embodiment 43, wherein the light absorbent bead is dyed with 5,5'-dichloro-11-diphenylamino-3,3'-diethyl-10,12-ethylenethiatricarbocyanine perchlorate (IR 140).

Embodiment 45 is the composition of any one of embodiments 28 to 44, wherein the antibody is an anti-platelet antibody.

Embodiment 46 is the composition of embodiment 45, wherein the antibody is an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody.

Embodiment 47 is the composition of any one of embodiments 28 to 46, wherein the composition is lyophilized.

Embodiment 48 is a method for quantifying platelets in a blood sample, wherein the method comprises (a) forming a mixture comprising (i) a blood sample and (ii) a composition comprising a solid substrate-antibody conjugate and one or more agents selected from the group consisting of protease inhibitors, inhibitors of platelet activation, stabilizing agents, and inhibitors of non-specific binding of the antibody, wherein the antibody has binding specificity for a platelet cell surface glycoprotein receptor, and incubated the mixture under conditions such that platelets in the blood sample bind to the antibody to generate solid substrate-antibody-platelet complexes; (b) subjecting the mixture to agglutination conditions, such that the solid substrate-antibody-platelet complexes agglutinate; (c) measuring agglutination of the solid substrate-antibody-platelet complexes; and (d) relating the measurement of step (c) to the number or concentration of platelets in the blood sample.

Embodiment 49 is an article of manufacture designed for point of care use, wherein the article of manufacture comprises: (a) a reagent for quantifying platelets in a blood sample, wherein the reagent comprises a composition comprising a solid substrate-antibody conjugate and one or more agents selected from the group consisting of protease inhibitors, inhibitors of platelet activation, stabilizing agents, and inhibitors of non-specific binding of the antibody, wherein the antibody has binding specificity for a platelet cell surface glycoprotein receptor; (b) a reagent for quantifying adenosine diphosphate (ADP) in the blood sample; (c) a reagent for quantifying thrombin receptor activating peptide (TRAP) in the blood sample; and (d) a reagent for quantifying arachidonic acid (AA) in the blood sample.

Embodiment 50 is an article of manufacture designed for point of care use, wherein the article of manufacture comprises: (a) a reagent for quantifying platelets in a blood sample, wherein the reagent comprises a composition comprising a solid substrate-antibody conjugate and one or more agents selected from the group consisting of protease inhibitors, inhibitors of platelet activation, stabilizing agents, and inhibitors of non-specific binding of the antibody, wherein the antibody has binding specificity for a platelet cell surface glycoprotein receptor; (b) a reagent for quantifying ADP in the blood sample; (c) a reagent for quantifying TRAP in the blood sample; and (d) a reagent for quantifying collagen in the blood sample.

Embodiment 51 is a method for generating a solid substrate-antibody conjugate, wherein the method comprises: (a) contacting a solid substrate comprising a polypeptide attached thereto with Traut's reagent to thiolate the polypeptide, thereby forming a solid substrate comprising a thiolated polypeptide attached thereto; (b) contacting an antibody with a heterobifunctional crosslinker comprising a maleimide reactive group, thereby forming a maleimide-activated antibody; and (c) combining (i) the solid substrate comprising the thiolated polypeptide attached thereto and (ii) the maleimide-activated antibody, wherein the maleimide-activated antibody becomes coupled to the solid substrate comprising the thiolated polypeptide attached thereto via a thiol-maleimide reaction, thereby forming the solid substrate-antibody conjugate.

Embodiment 52 is the method of embodiment 51, wherein the solid substrate is a bead.

Embodiment 53 is the method of embodiment 52, wherein the bead has a diameter of about 0.5 to about 10 µm.

Embodiment 54 is the method of embodiment 52, wherein the bead has a diameter of about 5.5 to about 6 µm.

Embodiment 55 is the method of any one of embodiments 51 to 54, wherein the bead comprises latex.

Embodiment 56 is the method of any one of embodiments 51 to 55, wherein the bead is a light absorbent bead.

Embodiment 57 is the method of embodiment 56, wherein the light absorbent bead is dyed with 5,5'-dichloro-11-diphenylamino-3,3'-diethyl-10,12-ethylenethiatricarbocyanine perchlorate (IR 140).

Embodiment 58 is the method of any one of embodiments 51 to 57, wherein the antibody is an anti-platelet antibody.

Embodiment 59 is the method of embodiment 58, wherein the antibody is an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody.

Embodiment 60 is the method of any one of embodiments 51 to 59, wherein the combining of the (i) and the (ii) is at a molar ratio such that the thiolated polypeptide is present at about a 20- to 80-fold molar excess as compared to the maleimide-activated antibody.

Embodiment 61 is the method of any one of embodiments 51 to 60, wherein the method further comprises lyophilizing the solid substrate-antibody conjugate.

Embodiment 62 is a solid substrate coupled to an antibody, wherein the solid substrate was coupled to the antibody using the method of any one of embodiments 51 to 61.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method for generating a composition comprising a solid substrate-antibody conjugate, wherein said method comprises:
(a) coupling an antibody to a solid substrate to generate a solid substrate-antibody conjugate; and
(b) adding one or more agents to said solid substrate-antibody conjugate, wherein said one or more agents are selected from the group consisting of (i) protease inhibitors, (ii) inhibitors of platelet activation, (iii) stabilizing agents, and (iv) inhibitors of non-specific binding of said antibody.

2. The method of claim 1, wherein said method comprises adding, to said solid substrate-antibody conjugate, one or more protease inhibitors, and/or
one or more inhibitors of platelet activation, and/or
one or more inhibitors of non-specific binding of said antibody.

3. The method of claim 1 or 2, wherein said solid substrate is a bead.

4. The method of claim 3, wherein said bead comprises latex.

5. The method of claim 3 or 4, wherein said bead is a light absorbent bead.

6. The method of any one of claims 1 to 5, wherein said antibody is an anti-platelet antibody.

7. The method of any one of claims 1 to 6, wherein said antibody is an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody.

8. A composition comprising:
a solid substrate-antibody conjugate; and
one or more agents selected from the group consisting of (i) protease inhibitors, (ii) inhibitors of platelet activation, (iii) stabilizing agents, and (iv) inhibitors of non-specific binding of said antibody.

9. The composition of claim 8, wherein said composition comprises one or more protease inhibitors, and/or
wherein said composition comprises one or more inhibitors of platelet activation, and/or
wherein said composition comprises one or more inhibitors of non-specific binding of said antibody.

10. The composition of claim 8 or 9, wherein said solid substrate is a bead.

11. The composition of 10, wherein said bead comprises latex, and/or
wherein said bead is a light absorbent bead.

12. The composition of any one of claims 8 to 11, wherein said antibody is an anti-platelet antibody.

13. The composition of any one of claims 8 to 12, wherein said antibody is an anti-platelet glycoprotein Ib (GP1b) antibody, an anti-platelet glycoprotein IIB/IIIa (GPIIB/IIIa) antibody, an anti-platelet glycoprotein 1b alpha chain (GPIBa) antibody, an anti-platelet glycoprotein IX (GPIX) antibody, or an anti-CD9 antibody.

14. The composition of any one of claims 8 to 13, wherein said composition is lyophilized.

15. A method for quantifying platelets in a blood sample, wherein said method comprises:
(a) forming a mixture comprising (i) a blood sample and (ii) a composition comprising a solid substrate-antibody conjugate and one or more agents selected from the group consisting of protease inhibitors, inhibitors of platelet activation, stabilizing agents, and inhibitors of non-specific binding of said antibody, wherein said antibody has binding specificity for a platelet cell surface glycoprotein receptor, and incubating said mixture under conditions such that platelets in said blood sample bind to said antibody to generate solid substrate-antibody-platelet complexes;
(b) subjecting said mixture to agglutination conditions, such that said solid substrate-antibody-platelet complexes agglutinate;
(c) measuring agglutination of said solid substrate-antibody-platelet complexes; and
(d) relating the measurement of step (c) to the number or concentration of platelets in said blood sample.
